(19)

Europäisches
Patentamt
European
Patent Office
Office européen
des brevets

(11) **EP 2 383 033 A1**

(12) **EUROPEAN PATENT APPLICATION**

(43) Date of publication:
02.11.2011 Bulletin 2011/44

(21) Application number: 10250776.1

(22) Date of filing: 14.04.2010

(51) Int Cl.:
$B01J\ 8/02^{(2006.01)}$   $C07C\ 17/154^{(2006.01)}$
$C07C\ 17/156^{(2006.01)}$   $C07C\ 19/01^{(2006.01)}$
$C07C\ 19/03^{(2006.01)}$   $C07C\ 19/045^{(2006.01)}$

(84) Designated Contracting States:
AT BE BG CH CY CZ DE DK EE ES FI FR GB GR
HR HU IE IS IT LI LT LU LV MC MK MT NL NO PL
PT RO SE SI SK SM TR
Designated Extension States:
AL BA ME RS

(71) Applicant: Stauffer, John E.
Greenwich
Connecticut 06830 (US)

(72) Inventor: Stauffer, John E.
Greenwich
Connecticut 06830 (US)

(74) Representative: Newell, Campbell et al
Marks & Clerk LLP
Aurora
120 Bothwell Street
Glasgow
G2 7JS (GB)

(54) **Swing reactor and process for oxychlorination**

(57)   A reactor with swing feeds is provided for oxychlorination. This reactor comprises multiple inlets with controls capable of introducing feed streams sequentially to the reactor. In one configuration, a feed stream comprises a paraffin or olefin hydrocarbon such as methane or ethylene, and a second feed stream comprises oxygen and hydrogen chloride. By segregating these feeds, combustion reactions can be minimized and yields of chlorinated components increased.

FIG. 3a

EP 2 383 033 A1

FIG. 3 b

**Description**

FIELD OF THE INVENTION

A catalytic oxychlorination process for the sequential feed of two or more streams of reactants is provided. One stream may comprise a paraffin or olefin hydrocarbon, and a second stream may comprise oxygen and hydrogen chloride. When these two streams are fed to the reactor containing an oxychlorination catalyst, the product will consist of a chlorinated component and water.

BACKGROUND

Numerous attempts have been made to oxychlorinate hydrocarbons, but the results have been disappointing. When methane, for example, is reacted with oxygen and hydrogen chloride over a suitable catalyst, methyl chloride and higher chlorinated methane compounds are produced. Unfortunately, under these reaction conditions, a significant amount of methane is purged to carbon monoxide and carbon dioxide.
To overcome this difficulty, various approaches have been tried. One strategy has been to identify an improved catalyst that will eliminate or at least reduce combustion. Another approach has been to focus attention on the reactor design. For example, a fluidized bed reactor has reputedly been investigated in order to provide better temperature control and presumably give higher yields of products.
Success in these ventures has proven to be elusive. Therefore, it is an object of the present invention to provide an improved reactor design for the oxychlorination of paraffin and olefin hydrocarbons. This object as well as other features and advantages will be apparent from the following description and the figures that are included.

SUMMARY

The present invention provides for a reactor and method of operation with swing feed to be used in the oxychlorination of paraffin and olefin hydrocarbons. Multiple streams of reactants are fed sequentially to the reactor, which contains an oxychlorination catalyst. Control of the feed streams is attained by actuating valves.
The paraffin hydrocarbon may be methane, propane, butane, ethane or any other alkane of interest. The olefin hydrocarbon may be methylene, ethylene or any other. These compounds may be reacted with hydrogen chloride and oxygen or air. Products may comprise mono-substituted or poly-substituted chlorinated hydrocarbons.
The catalyst employed may comprise salts of copper, iron and rare earths. Also, an alkali metal chloride may be incorporated into the catalyst to increase its activity. These catalyst components are deposited on an inert carrier to provide intimate contact with the gas phase.
Other applications of the present invention will become apparent to those skilled in the art when the following description of the best mode contemplated for practicing the invention is read in conjunction with the accompanying drawings.

BRIEF DESCRIPTION OF THE DRAWINGS

The description herein makes reference to the accompanying drawings wherein like reference numerals refer to like parts throughout the several views, and wherein:

FIGS. 1 a and 1 b are schematic diagrams of the swing reactor for the case in which two feed streams are employed;

FIGS. 2a, 2b and 2c are schematic diagrams for three feed streams; and

FIGS. 3a and 3b are swing reactor diagrams with two effluent streams.

DETAILED DESCRIPTION

In the prior art, oxychlorination of a hydrocarbon is conducted by feeding a single stream of reactants to a catalytic reactor. Such a single stream will have a uniform composition and contain a given paraffin, hydrogen chloride and oxygen all intimately mixed together.
In contrast to the accepted practice, the present invention contemplates the segregation of the hydrocarbon feed from the oxygen-containing stream. This feature is critical to suppress combustion. The segregation is accomplished by feeding the reactants sequentially in separate streams to the reactor.
Thus, in the specific case where the goal is to chlorinate methane or ethylene, first a stream containing oxygen and hydrogen chloride is fed to the reactor to regenerate the catalyst. After a predetermined time, this stream is turned off and a stream of methane or ethylene is fed to the reactor. Once the catalyst has been depleted of the available chlorine, the methane or ethylene system is shut off, and the cycle is repeated.

The chemistry that takes place during this sequence of events can be shown by the following equations.

$$2\ CuCl + 2\ HCl + 0.5\ O_2 \rightarrow 2\ CuCl_2 + H_2O \qquad (1)$$

where CuCl is cuprous chloride, HCl is hydrogen chloride, $O_2$ is oxygen, $CuCl_2$ is cuprous chloride, and $H_2O$ is water.

$$CH_4 + 2\ CuCl_2 \rightarrow CH_3Cl + HC1 + 2\ CuCl \qquad (2)$$

where $CH_4$ is methane, $CH_3Cl$ is methyl chloride.

The regeneration of the catalyst is shown in equation 1, and the depletion of the catalyst during the chlorination of methane is shown in equation 2. The chemistry for ethylene is shown by the following equations:

$$2\ CuCl + 2\ HCl + 0.5O_2 \rightarrow 2\ CuCl_2 + H_2O \qquad (3)$$

$$C_2H_4 + 2\ CuCl_2 \rightarrow C_2H_4Cl_2 + 2\ CuCl \qquad (4)$$

In equation (4), $C_2H_4$ is ethylene and $C_2H_4\ Cl_2$ is ethylene dichloride. It will be noted that equations (1) and (3) are the same.

The catalyst may contain other active constituents besides copper, but the chemistry is the same. For example, iron chloride may be included for the purpose of depressing the melting point of the catalyst. In this regard, potassium chloride is especially effective.

The reaction temperature must be sufficiently high to overcome the inertness of the alkane to be chlorinated. In the neighborhood of 450° the reaction kinetics are favorable, however, at these temperatures the alkane is subject to combustion. Therefore, the provisions of the present invention are all-important.

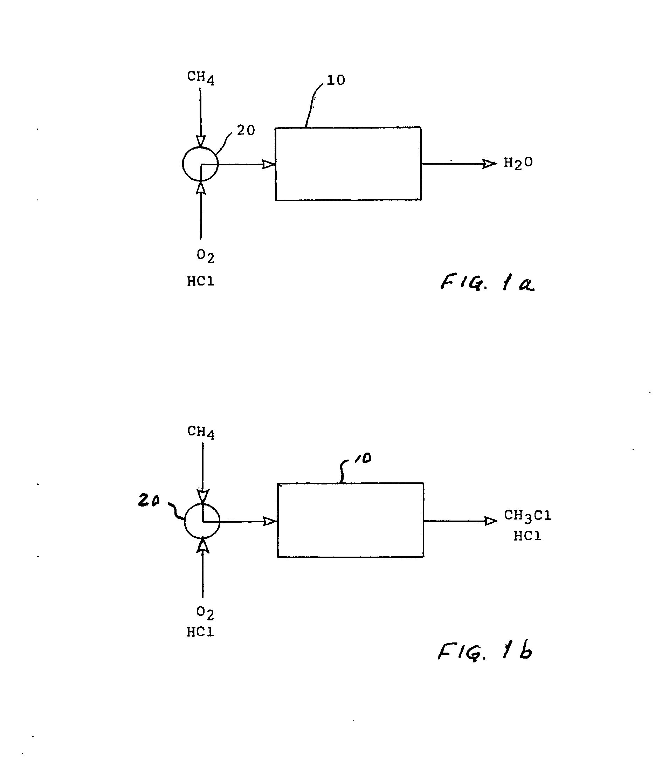

The actual workings of a swing reactor are illustrated in Fig. 1. First, the value 20 is set to provide the reactor 10 with a stream containing oxygen and hydrogen chloride as shown in Fig. 1a. Thereafter, by turning valve 20, the oxygen/hydrogen chloride stream is shut off and a stream of methane is fed to the reactor as shown in drawing Fig. 1 b.

Under ideal conditions, there is no mixing between the oxygen containing stream and the methane stream. Such a result can be achieved with plug flow, this condition can be approached by designing the reactor 10 to contain a minimum of dead space and by minimizing back mixing in the catalyst bed.

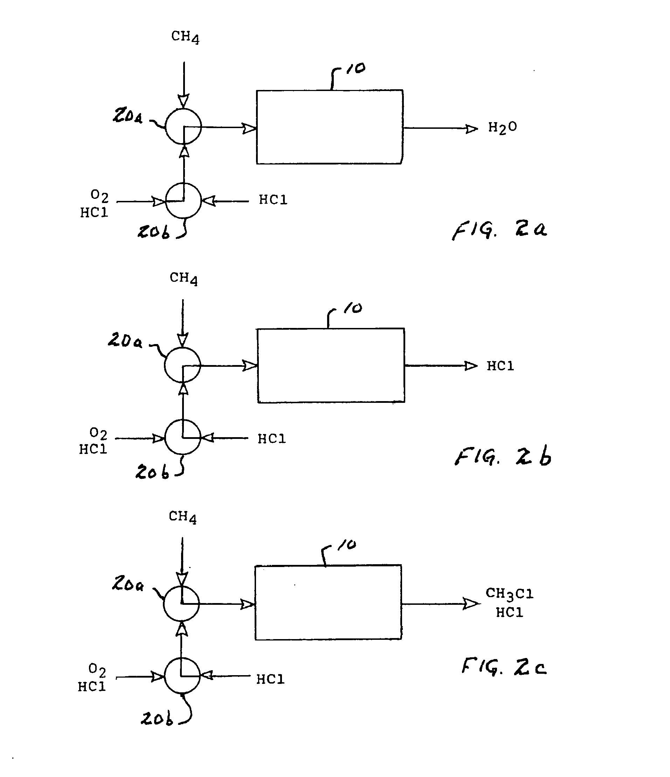

Figs. 2a, 2b and 2c show an alternative in which the hydrocarbon and oxygen streams are completely segregated. This reactor functions by using three feed streams: Fig. 2c, a methane (or ethylene) stream, Fig. 2a, an oxygen-containing stream, and Fig. 2b, a hydrogen chloride stream. Two valves, 20a and 20b, are required. The different modes of operating are shown in drawings (a), (b) and (c). A complete cycle of operation would contain the following sequence of feeds: oxygen, hydrogen chloride, methane, hydrogen chloride. The sequence then repeats.

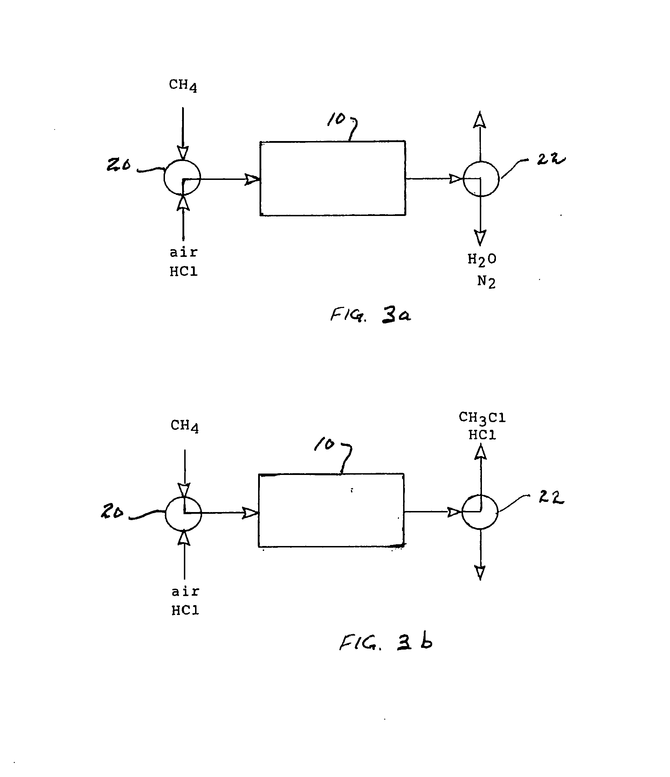

In chlorination processes, an excess of hydrocarbon feed is often employed in order to control the product yields. In these processes, the unreacted hydrocarbon is recovered and recycled to the feed stream. Such a procedure can be accommodated in a swing reactor by providing for two effluent streams as shown in Figs. 3a and 3b. Using this layout, air can be substituted for oxygen without complicating product recovery.

The input valve 20 is operated as described above with respect to Figs. 1 a and 1 b. Air, of course, contains oxygen as well as nitrogen, so the inputs to reactor 10 are essentially the same. An output valve 22 is added. One effluent stream is water and nitrogen while the other is CH3Cl and HCl, depending on the position of the valve.

The dynamics of a swing reactor depends on the relative quantity of catalyst and the flow rates. The cycle frequency can be expressed by the following equation.

$$(5) \qquad f = S/M$$

where f is the frequency in cycles per second, S is the flow rate of hydrogen chloride in moles per second, and M is the moles of copper chloride in the catalyst.

Some interesting conclusions can be drawn from the above expression. As the frequency is increased, the quantity of catalyst can be reduced for a given flow rate. Or keeping the catalyst constant, the flow rate can be increased by increasing the frequency.

The present invention represents an effective means of reducing and possibly eliminating combustion reactions during the oxychlorination of paraffin and olefin hydrocarbons. The result is increased yields of product, simplified recovery procedures, and lower capital investment. The flexibility provided by a swing reactor should expand the interest in oxychlorination chemistry.

While the invention has been described in connection with what is presently considered to be the most practical and preferred embodiment, it is to be understood that the invention is not to be limited to the disclosed embodiments but, on the contrary, is intended to cover various modifications and equivalent arrangements included within the spirit and scope of the appended claims, which scope is to be accorded the broadest interpretation so as to encompass all such modifications and equivalent structures as is permitted under the law.

## Claims

1. A catalytic reactor for the oxychlorination of a paraffin or olefin hydrocarbon, said reactor comprising multiple inlets with controls capable of introducing feed streams of the hydrocarbon and an oxychlorination reactant sequentially to the reactor.

2. A reactor according to claim 1 wherein the reactor is equipped with multiple outlets with controls capable of diverting product streams sequentially to different locations.

3. A reactor according to claim 1 wherein the paraffin hydrocarbon is methane or methylene.

4. A reactor according to claim 1 wherein the paraffin hydrocarbon is ethane or ethylene.

5. A reactor according to claim 1 wherein the paraffin hydrocarbon is propane.

6. A reactor according to claim 1 wherein the reactor contains a catalyst comprising a copper salt.

7. A method for the catalytic oxychlorination of a paraffin or olefin hydrocarbon comprising the separately and sequentially performed steps of:

   (a) feeding oxygen into a reactor containing an oxychlorination catalyst; and
   (b) feeding a paraffin or olefin hydrocarbon into said reactor wherein said hydrocarbon is an alkane.

8. A method as described in claim 7 wherein the alkane is one of methane, propane, butane, methylene and ethylene.

9. A method as described in claim 7 wherein the catalyst is chosen from the group consisting of salts of copper, iron and rare earths.

10. A catalytic reactor for the oxychlorination of a hydrocarbon comprising:

    a reactor containing an oxychlorination catalyst;
    means for introducing a hydrocarbon to the reactor; and
    means for separately and sequentially introducing oxygen to the reactor.

11. A catalytic reactor as described in Claim 1 wherein the means for introducing comprises a multi-position valve.

12. A catalytic reactor for the oxychlorination of ethylene, said reactor comprising multiple inlets with controls capable of sequentially introducing feed streams of said ethylene and oxychlorination reactants to the reactor.

CH₄

$$CH_4$$

O₂
HCl

FIG. 1a

CH₄

$$CH_4$$

O₂
HCl

$$CH_3Cl$$
$$HCl$$

FIG. 1b

CH$_4$

*20a*

O$_2$
HCl → *20b* ← HCl

*10*

→ H$_2$O

*FIG. 2a*

CH$_4$

*20a*

O$_2$
HCl → *20b* ← HCl

*10*

→ HCl

*FIG. 2b*

CH$_4$

*20a*

O$_2$
HCl → *20b* ← HCl

*10*

→ CH$_3$Cl
HCl

*FIG. 2c*

CH₄

20

air
HCl

10

22

H₂O
N₂

FIG. 3a

CH₄

20

air
HCl

10

CH₃Cl
HCl

22

FIG. 3b

8

Europäisches Patentamt
European Patent Office
Office européen des brevets

## EUROPEAN SEARCH REPORT

Application Number

EP 10 25 0776

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2009/312592 A1 (STAUFFER JOHN E [US]) 17 December 2009 (2009-12-17) * the whole document * ----- | 1-12 | INV.<br>B01J8/02<br>C07C17/154<br>C07C17/156<br>C07C19/01<br>C07C19/03<br>C07C19/045 |

TECHNICAL FIELDS SEARCHED (IPC)

B01J
C07C

The present search report has been drawn up for all claims

| Place of search | Date of completion of the search | Examiner |
|---|---|---|
| The Hague | 8 February 2011 | Delanghe, Patrick |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 2 383 033 A1**

## ANNEX TO THE EUROPEAN SEARCH REPORT
## ON EUROPEAN PATENT APPLICATION NO.

EP 10 25 0776

08-02-2011

| Patent document cited in search report | Publication date | Patent family member(s) | Publication date |
|---|---|---|---|
| US 2009312592 A1 | 17-12-2009 | NONE | |

EPO FORM P0459